# EUROPEAN PATENT APPLICATION

(11) **EP 3 246 414 A1**
(43) Date of publication of application: **22.11.2017**
(21) Application number: 16305582.5
(22) Date of filing: 20.05.2016
(51) Int. Cl.: C12Q 1/68

(54) **KIT AND METHOD FOR DETECTING OR QUANTIFYING ONE OR MULTIPLE NUCLEIC ACID TARGETS**

(71) Applicant: Universite Paris-Sud, 91400 Orsay (FR); Centre National de la Recherche Scientifique, 75016 Paris (FR); Lumiphore, Inc., Berkeley, CA 94710-2224 (US)
(72) Inventor: HILDEBRANDT, Niko, 91400 ORSAY (FR); QIU, Xue, 91120 PALAISEAU (FR)
(74) Representative: Pontet Allano & Associes

(57) **Abstract**

The present invention concerns a new kit for detecting or quantifying one or multiple nucleic acid targets in a biological sample by FRET measurement, and a detection or quantification method using this kit.

## Description

The present invention concerns a new kit for detecting or quantifying one or multiple nucleic acid targets in a biological sample, and a detection or quantification method using this kit.

Diagnostic detection technologies based on amplification of DNA are most often multistep and non-homogeneous. An exception is rolling circle amplification (RCA), in which an up to ca. 10,000 fold amplification can be achieved at a constant temperature. RCA can be coupled to the ligation of a padlock probe to provide sensitive detection of nucleic acids.

Up to now, most technologies for detecting RCA product use molecular beacon (MB) probes which are hairpin shaped molecules having a fluorophore and a black hole quencher on its two terminals (Wu et al., Anal. Chem. 2010, 82, 2221-2227). In the absence of RCA product as target, the fluorescence of the fluorophore is quenched by the black hole quencher because of a hairpin loop structure. In the presence of RCA, MB probes hybridize with it and restore the fluorescence. This technique provides a "turn-on" fluorescent signal to indicate the presence of an RCA product.

This technique suffers from both the high background from unquenched fluorescence signal in the closed conformation of MB and the decreased signal in the open conformation of MB due to the coiled structure of RCA products, which shortens the distance of fluorophore and quencher, and these aspects limit the sensitivity.

Another approach for detecting RCA products uses a pair of short linear DNA probes as signal probes, which are respectively labelled at the end by fluorophore and quencher (Ma et al., RSC Adv., 2015, 5, 4019). In the presence of RCA products as targets, thousands of fluorophore-labelled probes and quencher-labelled probes hybridize with the RCA product in head-to-head fashion on the coiled RCA product, thereby leading to fluorescence quenching. However, this "turn-off" mode is not preferred for developing new assays as it increases the standard deviation of the no-target sample. In fact, "signal off" techniques can create a high background of freely diffusing donor oligos that are not quenched. Even though this technique can avoid problems of the coiled RCA product structure, the increase in background fluorescence of freely diffusing donor fluorescence is too strong to enable this technique to be more sensitive than conventional "signal-on" techniques.

Furthermore, it is even less possible to detect multiple nucleic acid targets in one sample with high sensitivity by a conventional "signal-on" or "signal-off" technique.

There is therefore a need of providing a more sensitive method for detecting or quantifying, in a sample, at least one nucleic acid target or multiple nucleic acid targets amplified by RCA.

The first aspect of the present invention concerns a kit for detecting or quantifying at least one nucleic acid target of at least 16 nucleotides, in particular of 18 to 40 nucleotides, by time-resolved Förster resonance energy transfer (FRET) measurement combined with DNA-primed RCA, in a sample obtained from a biological sample, said nucleic acid target being represented from its 5' end to 3' end by structure TGT5'-TGT3', wherein
- TGT5' is the part situated at 5' end of a nucleic acid target;
- TGT3' is the part situated at 3' end of a nucleic acid target,
said kit being constituted by:
(i) at least one padlock probe of at least 60 nucleotides, in particular of 70 to 120 nucleotides, represented from its 5' end to 3' end by structure PRB5'-LINK-PRB3', wherein
   - PRB5' is a nucleic acid fragment of at least 8 nucleotides, in particular of 9 to 20 nucleotides, situated at 5' end of padlock probe, said PRB5' fragment having nucleic acid sequence complementary to that of at least a part of TGT5' fragment;
   - PRB3' is a nucleic acid fragment of at least 8 nucleotides, in particular of 9 to 20 nucleotides, situated at 3' end of padlock probe, said PRB3' fragment having nucleic acid sequence complementary to that of at least a part of TGT3' fragment;
   - LINK is a nucleic acid fragment situated between the fragments PRB5' and PRB3', of at least 44 nucleotides, in particular of 50-110 nucleotides,
      the padlock probe number being equal to that of nucleic acid target(s) to be detected or quantified,
(ii) at least one pair of energy donor-acceptor probes, whose number is equal to that of nucleic acid target(s), in each pair:
   - said energy donor probe named D-probe comprising an oligonucleotide named Nd-D of at least 8 nucleotides, in particular 10 to 30 nucleotides, Nd-D having nucleic acid sequence identical to that of a first part of padlock probe, said Nd-D oligonucleotide being labelled at 5' or 3' end by a luminescent material of long excited-state lifetime, as energy donor,
   - said energy acceptor probe named A-probe comprising an oligonucleotide named Nd-A of at least 8 nucleotides, in particular 10 to 30 nucleotides, Nd-A having nucleic acid sequence identical to that of a second part of padlock probe, said Nd-A oligonucleotide being labelled at 5' or 3' end by a luminescent material, as energy acceptor,
   - said energy donor having an average excited-state lifetime at least 10 times longer than any one of the energy acceptor,
(iii) optionally, a ligase,
(iv) optionally, a polymerase,
(v) optionally, reaction buffers.

The technique proposed by the present invention is based on nucleic acid target primed RCA, which is combined, for the first time, with time-resolved FRET from a long excited-state lifetime energy donor to an acceptor. Against all odds, the special and optimized combinations of donor-acceptor pair and donor-acceptor distance enable to decrease background fluorescence during RCA product detection, which can be caused by directly-excited acceptor fluorescence, and improve therefore the target nucleic acid detection sensitivity.

The sensitivity provided by the kit of the present invention was shown to reach a limit of detection of 5 fM and is not affected by the coil structure of the RCA product. Theoretically, even lower limits of detection are possible. This technique offers a "turn-on" signal based on the ratiometric measurement, which means that the ration of time-gated photoluminescence (PL) intensities of the acceptors and the donors are used as quantifying signal.

Moreover, this technique can discriminate more than one nucleic acid target by performing color (or spectral) multiplexing using fluorophores with different emission bands and/or temporal multiplexing based on different excited-state lifetimes of one or several fluorophores.

Rolling-Circle Amplification (RCA) is an isothermal, enzymatic process mediated by DNA polymerases, in which long single-stranded DNA molecules (RCA product) are synthesized on a short circular single-stranded template by using a single DNA primer.

During the process of RCA, a nucleic acid target to be detected can serve as DNA primer and amplify up to 10, 000- fold in a few hours at a constant temperature , for example in room temperature of from 15°C to 37°C.

In the present invention, said circular DNA template is formed by a padlock probe after junction of its 5'- end with its 3'- end by a ligase.

The term "padlock probe" means a single-stranded linear probe of at least 60 nucleotides, in particular of 60-300 nucleotides, more in particular 70 to 120 nucleotides. A padlock probe contains a fragment PRB5' at its 5'-end and a fragment PRB3' at its 3'-end, which respectively hybridize with a single-stranded nucleic acid target in a juxtaposed manner. In another word, the 5'-end part of a padlock probe hybridizes with the 5'-end part of a nucleic acid target and the 3'-end part of said padlock probe hybridizes with the 3'-end part of said nucleic acid target. After hybridization with the target and in the presence of a ligase, the phosphorylated 5'-end of padlock probe is jointed to the 3'-end of said padlock.

After the ligation of the padlock probe, said nucleic acid target, which uses the circular padlock probe as template, is amplified in the presence of DNA polymerase and dNTP.

The ligase in a kit of the present invention can be any DNA or RNA ligase known in prior art, which can catalyse the formation of a phosphodiester bond, in particular the T4 DNA ligase.

The polymerase in a kit of the present invention can be any DNA polymerase known in prior art, in particular the Phi29 DNA Polymerase.

The term "nucleic acid target" includes DNA or RNA. Said nucleic acid target may be either a single-stranded nucleic acid or a double-stranded nucleic acid. Examples of nucleic acid target can include microRNAs, siRNAs, single-stranded DNAs, DNA type or RNA type synthetic oligonucleotides, cDNAs, PCR amplified products, or fragments of genomic DNA or of mRNA, or a mixture thereof.

By "oligonucleotide" is meant a short single-stranded DNA or RNA of 5-50 nucleotides. An oligonucleotide used in the present invention may be a synthetic oligonucleotide, whose sequence can be designed with the help of conventional sequence design software.

In an embodiment of the present invention, the whole nucleic acid sequence of PRB5' fragment is complementary to a part of the sequence of TGT5'; and the whole nucleic sequence PRB3' fragment is complementary to a part of the sequence of TGT3'. In another word, the length of PRB5' and/or PRB3' fragment can be respectively shorter than that of TGT5' and/or TGT3' fragment. This is particularly the case when the nucleic acid target is a long genomic DNA.

In another embodiment of the present invention, the nucleic acid sequence of PRB5' fragment is completely complementary to that of TGT5' of a nucleic acid target to be detected or quantified; the nucleic acid sequence of PRB3' fragment is completely complementary to that of TGT3' of the same nucleic acid target.

According to the present invention, RCA products obtained from amplification of a nucleic acid target are detected with the help of one pair of energy donor-acceptor probes (D-probe and A-probe). Said pair of probes hybridize with the RCA product in adjacent regions through Nd-D and Nd-A oligonucleotides. Among Nd-D and Nd-A oligonucleotides of a pair of probes, which are designed for detecting a certain RCA product, at least one of said two oligonucleotides hybridizes exclusively with said RCA product, which ensures the specificity of each pair of probes.

Förster resonance energy transfer (FRET) is a mechanism of energy transfer between two light-sensitive molecules. An energy donor molecule, initially in its electronic excited state, may transfer energy to an energy acceptor molecule through nonradiative dipole-dipole coupling.

By "energy donor" is meant a material which, in its electronic excited state, may transfer energy to an energy acceptor. An energy donor may be excited by an exterior energy, such as the light, chemical reaction, biochemical reaction, electricity, magnetism, mechanics or fraction.

By "energy acceptor" is meant a material, which can physically be excited by the energy transferred from an energy donor.

In accordance with the present invention, in order to enable FRET, the absorption spectrum of energy acceptor is at least partially overlapped by the emission spectrum of energy donor.

Another condition for enabling FRET is the distance between the energy donor of D-probe and the energy acceptor of A-probe.

This distance determines the energy transfer efficiency, which is proportional to the distance to the power of -6. In accordance with the present invention, the FRET efficiency can be adjusted by changing donor-acceptor distance, which is typically between ca. 1 and 15 nm.

In a pair of D-probe and A-probe of the present invention, the energy donor and the energy acceptor can be both respectively situated on the 5' end of D-probe and A-probe, or both on the 3' end of D-probe and A-probe, or on opposed ends of said two probes.

Consequently, the distance between the energy donor and the energy acceptor can be modulated by the length of Nd-D oligonucleotide and/or that of Nd-A oligonucleotide, and eventually by the length of oligonucleotide which separates the D-probe and the A-probe after their hybridization with RCA product as target.

In the present invention, another criteria for the choice of donor-acceptor pair is that the excited-state lifetime of energy donor is at least 10 times longer than that of the energy acceptor.

This difference between excited-state lifetime of energy donor and that of energy acceptor enables the time-resolved FRET (TR-FRET) or time-gated FRET (TG-FRET), in another words, the measurement of the energy transfer based acceptor fluorescence emission after a time delay during which the energy acceptor direct excitation fluorescence signal has remarkably decayed. Necessary delay time in TG-FRET measurement is a function of the fluorescence lifetime of directly excited energy acceptor.

The FRET-sensitized fluorescence decay time of energy acceptor is determined, on the one hand, by the distance between the energy donor and the energy acceptor and, on the other hand, by the absorbance properties of each energy acceptor.

In accordance with the present invention, the energy donor is chosen from the group comprising a luminescent lanthanide ion or a transition metal such as Ru, Ir, Os, Pt or Re, or a long-lifetime fluorophore such as fluorazaphores (Hötzer et al., 2012, Small 8, No. 15, 2297-2326), or a long-lifetime nanoparticle such as semiconductor quantum dots or quantum rods or a combination thereof.

In preferred embodiment, the energy donor is a lanthanide ion chosen from Tb³⁺, EU³⁺, Sm³⁺, Dy³⁺, Er³⁺, Tm³⁺ or Ho³⁺.

According to a preferred embodiment, said luminescent material as energy donor is Tb³⁺, or in particular a Lumi4®-Tb complex, which has an extremely long excited-state lifetime of about 2.7 ms. Lumi4®-Tb can be efficiently excited in the UV (e.g., 337 nm emission of a nitrogen laser), which leads to mainly four bright and narrow photoluminescence (PL) emission bands in the 475 nm to 640 nm wavelength range.

By "quantum dot" is meant an inorganic semiconductor nanoparticle, which could include an organic coating, having a diameter from 1 nm to 100 nm, particularly from 5 nm to 40 nm.

By "quantum rod" is meant a rod shaped semiconductor nanocrystal, which could include an organic coating, with diameters ranging from 2 to 15nm and with lengths ranging from 5 to 200nm.

The quantum dots or quantum rods used in the present invention can be any long-lifetime quantum dot or quantum rods described in prior art, such as Cd-based or In-based quantum dots or quantum rods, in particular CdSe-ZnS core-shell nanoparticles with an additional organic coating.

The energy acceptor is an organic fluorophore, such as pyrene-, naphthalene-, coumarin-, fluorescein-, rhodamine- or cyanine-based dyes, or a non-fluorescent dark quencher, or a polymeric or dendrimeric dye, or a nanoparticle such as semiconductor quantum dot or quantum rod, or a naturally occurring fluorophore such as fluorescent protein or light harvesting complex or a combination thereof.

A "non-fluorescent dark quencher" refers to a material which does not emit fluorescence in excited-state. Examples of non-fluorescent dark quencher to be cited are dabsyl (dimethylaminoazobenznesulfonic acid), BBQ650 or BHQ-2 (The Molecular Probes® Handbook, Thermo Fisher Scientific).

As fluorescent protein, for example GFP, EGFP, YFP, CFP, BFP, dsRed, and mCherry (and derivates of the aforementioned) can be mentioned.

By "light harvesting complex" is meant a light collecting protein such as phycobili proteins, in particular b-phycoerythrin, R-phycoerythrin, and allophycocyanin and derivates thereof.

In a particular embodiment, when the energy donor is semiconductor quantum dots or quantum rods or a combination thereof, the energy acceptor is chosen from an organic fluorophore, or a non-fluorescent dark quencher, or a polymeric or dendrimeric dye, or a naturally occurring fluorophore such as fluorescent protein or light harvesting complex or a combination thereof.

A particular aspect of the kit of the present invention is that it can be used for detecting or quantifying with improved sensitivity more than one nucleic acid target, so-called multiplexing.

According to the present invention, a kit for detecting or qualifying more than one nucleic acid target contains more than one target-specific padlock probe for amplifying a specific RCA product for each target, the number of padlock probes being equal to that of nucleic acid targets, and the same number of pairs of energy donor-acceptor probes for detecting obtained RCA products.

Preferably, when said kit contains more than one target-specific padlock probe, the LINK fragment of each target-specific padlock probe has an exclusive region whose sequence is specific for each padlock probe to ensure target-specific hybridization between RCA product and each pair of energy donor-acceptor probes; each pair of energy donor-acceptor probes comprises, on its Nd-D or Nd-A oligonucleotide, an exclusive region whose sequence is identical to abovementioned exclusive region of padlock probe.

Different nucleic acid targets can be discriminated by a kit of the present invention through color (or spectral) multiplexing or temporal multiplexing, or a combination of thereof.

The term "Color multiplexing" refers to a multiplexing based on distinguishable emission spectra of energy acceptors and donors. In the present invention, this term is exchangeable with the term "spectral multiplexing". (Qiu and Hildebrandt. Rapid and Multiplexed MicroRNA Diagnostic Assay Using Quantum Dot-Based Förster Resonance Energy Transfer. ACS Nano 2015, 9(8), 8449-8457; Jin et al.,. Rapid, Amplification-Free, and Sensitive Diagnostic Assay for Single-Step Multiplexed Fluorescence Detection of MicroRNA. Angewandte Chemie International Edition 2015, 54, 10024-10029.)

A color multiplexing can be performed when each used energy acceptor has distinguishable emission signal spectra. Such a distinction can be done, for example, by mathematical deconvolution of the different spectra or by the use of appropriate optical bandpass filters. The spectra need to be sufficiently different in their spectral position, which is known by a person skilled in the art. For example, the intensity maxima of two different spectra must be separated by at least 5 nm.

According to an embodiment, the kit of the present invention contains at least two padlock probes and at least two pairs of energy donor-acceptor probes, wherein:
- the energy donors of each energy donor probe are identical; and
- the energy acceptors of each energy acceptor probe are different from each other.

In a preferred embodiment, there is no overlapping of emission spectra within the optical bandpass filters between the acceptors of each pair of energy donor-acceptor probes of the kit of the present invention.

According to a particular embodiment, the kit of the present invention contains three pairs of D-probe and A-probe, wherein the energy donor of each D-probe is Tb³⁺, or in particular a Lumi4®-Tb complex; the energy acceptors of three A-probes are respectively dye Cy3.5, Cy5 and Cy5.5. This kit can be used for detecting or quantifying three nucleic acid targets. Tb has four bright and narrow photoluminescence (PL) emission bands, which overlap with the absorbance spectra of all these three dyes.

By "temporal multiplexing" is meant a method of transmitting and receiving independent signals over a common signal path by means of synchronized switches at each end of the transmission line so that each signal appears on the line only a fraction of time in an alternating pattern.

Temporal multiplexing can be operated when the emission signal of different energy acceptors and/or donors can be detected in different time delays or time windows after the excitation pulse.

This difference of emission signal delay can be modulated through choosing energy acceptors having different absorbance properties or through changing the distance or orientation of donor-acceptor probes.

According to another embodiment, the kit of the present invention contains at least two padlock probes and at least two pairs of energy donor probe and energy acceptor probe, wherein
- the energy donors of each energy donor probe are identical;
- the energy acceptors of each energy acceptor probe are also identical ;
- the distances between the energy donor and the energy acceptor of each pair of energy donor-acceptor probes are different.

By "the distance between the energy donor and the energy acceptor of each pair of energy donor-acceptor probes" is meant the donor-acceptor distance after that D-probe and A-probe have been hybridized with RCA product.

This distance can be modulated by the length of Nd-D oligonucleotide and/or that of Nd-A oligonucleotide, and/or the length of oligonucleotide, which separates the D-probe and the A-probe after their hybridization with RCA product as target.

Preferably, the difference of energy donor-acceptor distance for each pair of energy donor-acceptor probes is at least 1 nm.

The kit of the present invention can be used for detecting or quantifying at least one nucleic acid target in a sample obtained from biological fluid, from an *in vitro* cell culture, from an *ex vivo* tissue, from plants, from yeast, from bacteria or from exosomes.

By "biological fluid" is meant a liquid contained, excreted or secreted from a living animal or plant, for example: blood, different fractions of blood, lymph, bile, saliva, exudates. In a preferred embodiment of the present invention, the biological fluid is a human or animal origin fluid chosen from serum, inactivated serum, plasma, or blood.

By "tissue" is meant a human, animal or vegetal tissue. In a particular embodiment of the invention, the sample of a tissue is a sample obtained by biopsy or during surgical operation. In a more particular embodiment, the tissue is a tumoral tissue obtained by biopsy or during surgical operation from a patient suffering from a cancer, or suspected to develop a cancer.

Another subject-matter of the present invention is also to provide a method for detecting or quantifying the amount of one or multiple nucleic acid targets of at least 16 nucleotides, in particular 18 to 40 nucleotides, in a sample obtained from a biological sample.

Said method comprises the steps of:
(i) Adding to said sample or a solution extracted from said sample, the padlock probe(s) of the kit as defined in claim 1, a ligase and a ligation buffer,
(ii) Adding to the mixture obtained at the end of step (i) a polymerase, dNTP and polymerase buffer to carry out a rolling circle amplification,
(iii) Adding to the mixture obtained at the end of step (ii), the pairs of energy donor-acceptor probes, whose number is equal to that of padlock probes(s),
(iv) Measuring photoluminescence emission intensities issued from energy donor and energy acceptor in a specific time window.

The step (i) of the method of the present invention ensures that the 5' and 3' ends of padlock probe is jointed and ligated after hybridizing with the nucleic acid target.

The ligase used in the step (i) can be any DNA or RNA ligase known in prior art which can catalyse the formation of a phosphodiester bond, in particular the T4 DNA ligase.

The rolling circle amplification of step (ii) can be carried out by any polymerase known in prior art, in particular the Phi29 DNA Polymerase.

Typically, the ligation and the RCA is carried out at ambient temperature, especially at 37°C.

The photoluminescence emission intensity measurements can be carried out by any conventional instrument, such as fluorescence spectrometers or fluorescence microscopes. In the case of time-resolved or time-gated PL measurements time-resolved detectors such as photomultiplier tubes (PMTs) or intensified CCD (ICCD) cameras or other time-gated detectors are necessary.

According to the method of the present invention, the photoluminescence emission intensities issued from energy donor and acceptor are measured in a specific time window starting at a time point of at least 10 times of the excited-state lifetime of the energy acceptor after light excitation and having a temporal width of at least the excited-state lifetime of the energy acceptor.

In a particular embodiment, the method of the present invention comprises further a step (v) after the step (iv), of comparing photoluminescence emission intensities obtained in previous step with pre-established standard intensities to determine the amount of nucleic acid target(s).

Pre-established standard intensities (so-called assay calibration curves) are determined from a series of samples containing all the probes of the kit according to the invention and different defined concentrations of the different nucleic acid targets.

In a particular embodiment, the present invention concerns a method for detecting or quantifying the amount of at least two nucleic acid targets in a sample, wherein at least two padlock probes and at least two pairs of energy donor-acceptor probes are used,
- the energy donors of each energy donor probe being identical;
- the energy acceptors of energy acceptor probe being also identical,
the photoluminescence emission intensity issued from each energy acceptor is measured in distinct specific time windows after light excitation.

In a preferred embodiment, there is no overlapping of time windows for measuring PI emission intensity of each energy acceptor.

In a particular embodiment, the time windows for each energy acceptor are separated by at least 1 ms.

In another particular embodiment, when the nucleic acid target to be determined or quantified in a sample is a double-stranded DNA, said method of the invention comprises before the step (i) a step for denaturing double-stranded nucleic acid and preparing single strand nucleic acid target.

In a particular embodiment, the method of the present invention is used for diagnosis or prognosis of a disease, such as a cancer or cardiovascular diseases.

In another embodiment, the method of the present invention is also used for *in vitro* diagnosis or prognosis of a disease, such as a cancer or cardiovascular diseases.

Particularly, the kit of the present invention can be used for detecting the presence of single nucleotide polymorphism (SNP) sites in a fragment of genomic DNA.

The present invention is illustrated in detail in following figures and examples.

### Figures

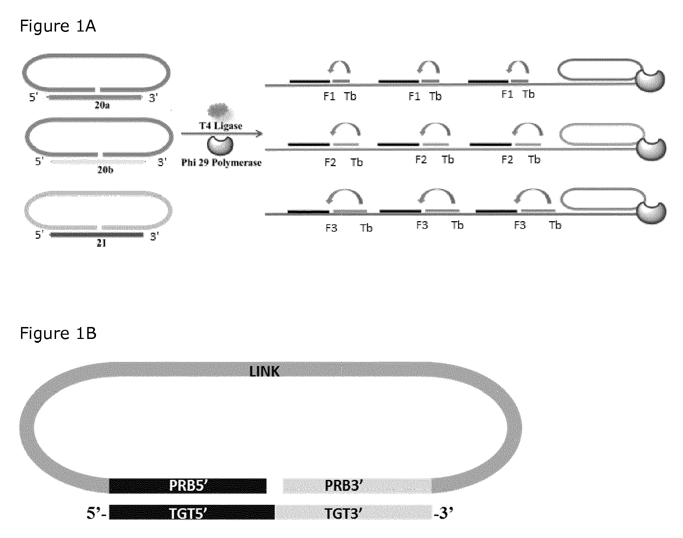

Figure 1A is a scheme showing the method of the present invention. ssDNA/RNA serves as the primer and after the hybridization with padlock probe, the nick is ligated by DNA or RNA ligase. After adding the polymerase and dNTP, the ssDNA/RNA can be amplified thousands of times in a few hours. After the generation of RCA products luminescent terbium complex Lumi4-Tb (Tb) labeled oligonucleotides and fluorophores labeled oligonucleotides (F) according to RCA products will hybridize with certain locations of RCA products and the detection is based on time-gated Förster resonance energy transfer from the Tb to fluorophores. To detect several targets in a single sample (multiplexing) the use of different fluorophores (F1 to F3 in the scheme) will allow for color multiplexing and the use of different distances between Tb and F (as indicated by the curved arrows) and/or different fluorophores will allow for temporal multiplexing. Both multiplexing approaches can also be combined for the creation of many different optical barcodes.
Figure 1B illustrates the structure of a padlock probe (PRB5'-LINK-PRB3') and that of a nucleic acid target (TGT5'-TGT3').
Figures 2A and 2B illustrate photophysical properties of the FRET sensor.

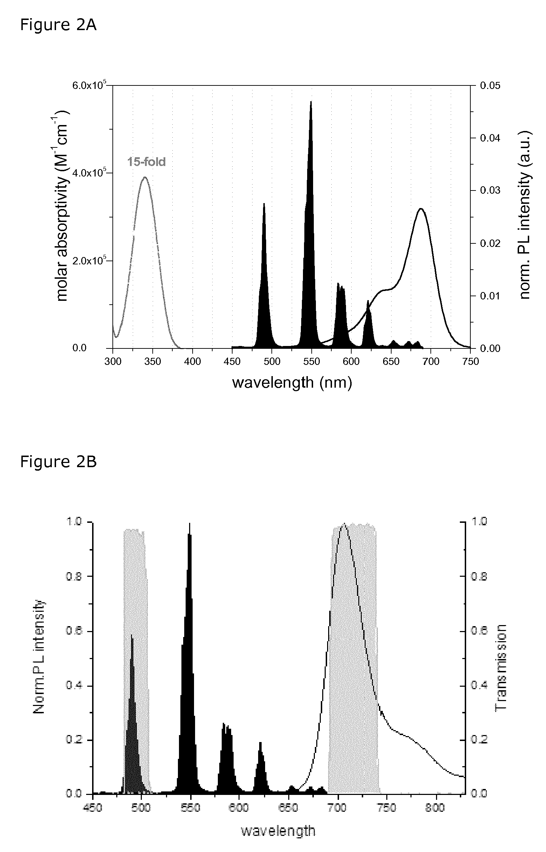

Figure 2A: Absorbance spectra of the probe-oligos containing Tb-donor (grey dotted curve) and the acceptor dye Cy5.5 (black curve). Area-normalized PL spectrum of Tb (black curve with black background) is shown for visualization of donor-acceptor spectral overlap. Förster distance was 5.8 nm for Tb-to-Cy5.5 FRET. Figure 2B: PL emission spectra of Tb (black curve with black background) and Cy5.5 (black curve). Optical transmission filter bandpass wavelengths were (494±10) nm (Semrock) for the Tb detection channel and (716±20) nm (Semrock) for the Cy5.5 detection channel. For color (or spectral) multiplexing, different acceptors, e.g. Cy3.5, Cy5 and Cy5.5 (as shown in Jin et al., Angewandte Chemie International Edition 2015, 54, 10024-10029) or 3 different quantum dots (as shown in Qiu and Hildebrandt. ACS Nano 2015, 9(8), 8449-8457) can also be used.

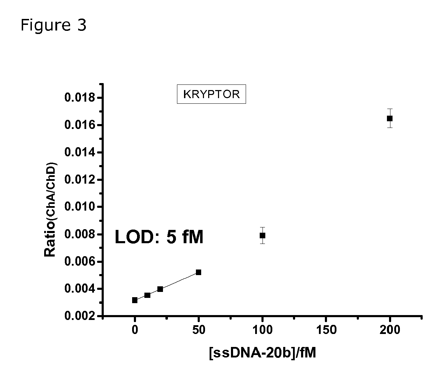

Figure 3 shows the FRET ratios (ratios of 0.1 - 0.9 ms time-gated PL intensities of Cy5.5 and Tb) as a function of ssDNA-20b concentration. For statistical analysis and the estimation of LODs all samples were prepared 3 times and measured in triplicates (n = 9) on a KRYPTOR clinical plate reader, apart from the zero-concentration samples (without ssDNA-20b targets (SEQ ID NO: 2)), which were prepared 10 times and measured in triplicates (n = 30). For the quantification of multiple targets by color (or spectral) multiplexing, different acceptors, e.g. Cy3.5, Cy5 and Cy5.5 (as shown in Jin et al., Angewandte Chemie International Edition 2015, 54, 10024-10029) or 3 different quantum dots (as shown in Qiu and Hildebrandt. ACS Nano 2015, 9(8), 8449-8457) can also be used.

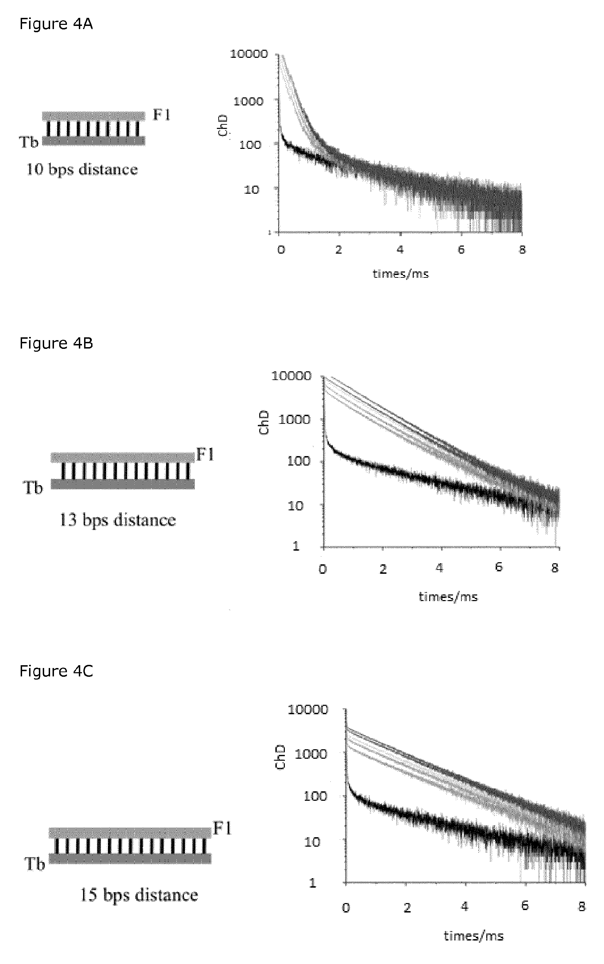

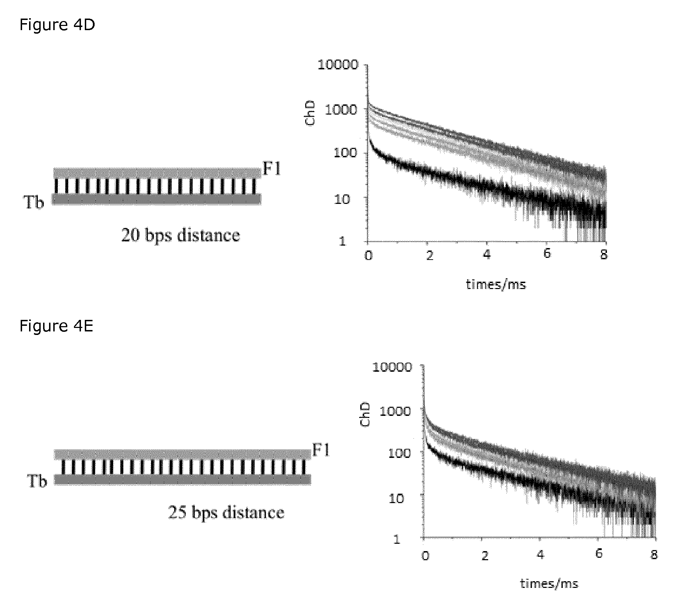

Figures 4A, 4B, 4C, 4D, and 4E illustrate temporal multiplexing using a Tb donor and a dye acceptor F1. The acceptor fluorescence decay curves of the same Tb-dye (Tb-F1) donor-acceptor pair separated by different distances (10, 13, 15, 20, and 25 base pairs of double-stranded DNA formed by a ssDNA labelled at 5' end by Tb and another ssDNA labelled at 5' end by F1 through DNA hybridization) show distinct temporal decays (shortest to longest from top to bottom). The black curves are pure Tb PL and the different gray curves represent acceptor PL with different concentrations. The distance of 10 base pairs is produced by ssDNA-Tb probe of SEQ ID NO: 11 and ssDNA-F1 probe of SEQ ID NO: 16. The distance of 13 base pairs is produced by ssDNA-Tb probe of SEQ ID NO: 12 and ssDNA-F1 probe of sequence SEQ ID NO: 17. The distance of 15 base pairs is produced by ssDNA-Tb probe of SEQ ID NO: 13 and ssDNA-F1 probe of SEQ ID No: 16. The distance of 20 base pairs is produced by ssDNA-Tb probe of SEQ ID NO: 14 and ssDNA-F1 probe of SEQ ID NO: 16. The distance of 25 base pairs is produced by ssDNA-Tb probe of SEQ ID NO: 15 and ssDNA-F1 probe of SEQ ID NO: 18.

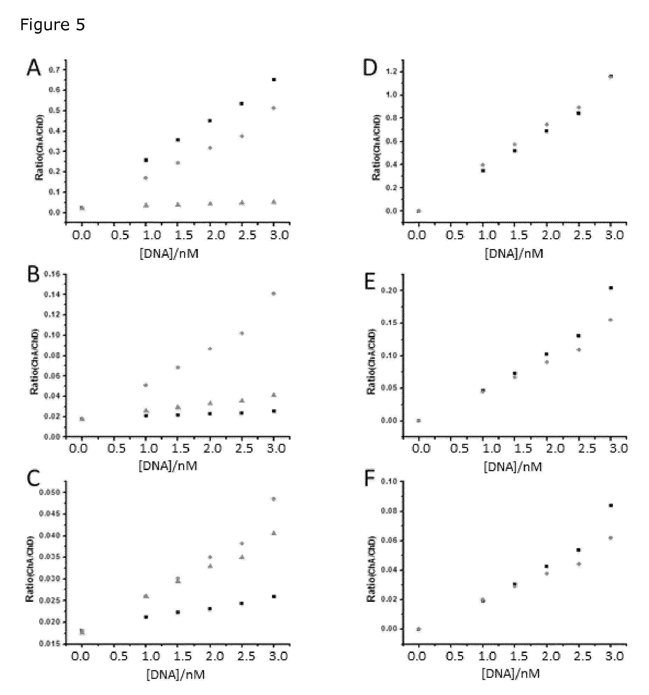

Figures 5A, 5B, 5C, 5D 5E and 5F: Triplexed ssDNA-F1 detection using the same Tb-F1 FRET pair separated by 10, 13 and 25 base pairs. Figures 5A, 5B and 5C show FRET signals of the 10 based (black squares), 13 bases (gray rhombi), and 25 bases (gray triangles) separated FRET pairs in three time-gated windows (Figure 5A: TGi: 0.02-1.5 ms, Figure 5B: TGii: 2.5-5.0 ms, Figure 5C: TGiii: 6.0-8.0 ms). Figures 5D, 5E and 5F show that the FRET ratios of the samples when all three F1 probes were present (black square) overlaid with the FRET ratios summation calculated of the samples when only one of the F1 probes was present (gray rhombi) in the same time-gated windows (Figure 5D: TGi: 0.02-1.5 ms, Figure 5E: TGii: 2.5-5.0 ms, Figure 5F: TGiii: 6.0-8.0 ms).

### Example

### 1. Materials and methods

### 1.1 Materials

Lumi4^{®}-Tb-NHS was provided by Lumiphore, Inc. All oligonucleotides were purchased from Eurogentec. Non-modified oligonucleotides were purified with cartridge gold desalting method. Phosphate oligonucleotides were purified with polyacrylamide gel electrophoresis. All other modified oligonucleotides were purified with HPLC. Water was purified by Purelab Option-Q equipped with bio-filter (ELGA LabWater). All chemicals were used as received. Tris(hydroxymethyl)-aminomethane and (NH₄)₂SO₄ were purchased from Sigma-Aldrich. NaCl, MgCl₂ and KCl were purchased from Duchefa (The Netherlands). T4 DNA ligase was purchased from Fermentas. Phi29 polymerase, ATP and dNTP mix were purchased from Thermo Fisher Scientific. The sequences of nucleic acid targets (ssDNA-20a, ssDNA-20b, ssDNA-21), those of padlock probes, those of Nd-D oligonucleotides of D-probes and of Nd-A oligonucleotide of A-probe are summarized in **Table 1**.

**Table 1. Oligonucleotide properties**

| Probe | Sequence 5'-3' | Modification |
|---|---|---|
| ssDNA-20a | TAAAGTGCTTATAGTGCAGGTAG (SEQ ID NO : 1) | / |
| ssDNA-20b | CAAAGTGCTCATAGTGCAGGTAG (SEQ ID NO : 2) | / |
| ssDNA-21 | TAGCTTATCAGACTGATGTTGA (SEQ ID NO : 3) | / |
| padlock ssDNA-20a | AAG CAC TTT AAT ATA TGA TGG ACC GCT CAA TTA ACT GAA GAT GCC GAA TTT TTC AAG AGG AAA TGT CTA CCT GCA CTA T (SEQ ID NO : 4) | 5'-Phosphate |
| padlock ssDNA-20b | GAG CAC TTT GAT ATA TGA TGG ACC GCT AGG ATT AAC TGA GAT GCC GAA TTT TTC AAG AGG AAA TGT CTA CCT GCA CTA T (SEQ ID NO : 5) | 5'-Phosphate |
| padlock ssDNA-21 | TGA TAA GCT AAT ATA TGA TGG AAT CAA GAC AAT ATT GTT GAT GCC GAA TTT TTC AAG AGG AAA TGT TCA ACA TCA GTC (SEQ ID NO : 6) | 5'-Phosphate |
| Cy5.5 probe | GATGCCGAATTTTTCAAGAG (SEQ ID NO : 7) | 5'- Cy5.5 |
| Tb probe ssDNA-20a(10 bps, Tm 24) | ATTAACTGAA (SEQ ID NO : 8) | 5'- C6 amino |
| Tb probe ssDNA-20b(13 bps, Tm 34) | TAGGATTAACTGA (SEQ ID NO : 9) | 5'- C3 amino |
| Tb probe ssDNA-21 (18 bps, Tm 44) | AATCAAGACAATATTGTT (SEQ ID NO : 10) | 5'- C6 amino |
| ssDNA-Tb (10 bps) | ATTCGGCATC (SEQ ID NO : 11) | 5'- C3 amino |
| ssDNA-Tb (13 bps) | TAGGATTAACTGA (SEQ ID NO : 12) | 5'- C3 amino |
| ssDNA-Tb (15 bps) | GAAAAATTCGGCATC (SEQ ID NO : 13) | 5'- C3 amino |
| ssDNA-Tb (20 bps) | CTCTTGAAAAATTCGGCATC (SEQ ID NO : 14) | 5'- C3 amino |
| ssDNA-Tb (25 bps) | TCGTGTCTAAAGTCCGTTAGCTCAT (SEQ ID NO : 15) | 5'- C3 amino |
| ssDNA-F1 (10 bps, 15 bps, 20 bps) | GATGCCGAATTTTTCAAGAG (SEQ ID NO : 16) | 5'- F1 |
| ssDNA-F1 (13 bps) | TCAGTTAATCCTA (SEQ ID NO : 17) | 5'- F1 |
| ssDNA-F1 (25 bps) | ATGAGCTAACGGACTTTAGACACGA (SEQ ID NO : 18) | 5'- F1 |

### 1.2 Methods and Results

***Tb-DNA conjugates.*** Tb-DNA conjugates were obtained by mixing Lumi4^{®}-Tb-NHS (lyophilized product dissolved to 8 mM in anhydrous DMF) in concentration excess to amino-functionalized oligonucleotides in 100 mM carbonate buffer at pH 9.0. The mixtures were incubated while rotating at 30 RPM (Intelli-Mixer, ELMI) overnight at room temperature. The Tb-DNA conjugates were purified 3 times by Zeba Spin Desalting Columns (7 kDa MWCO). Tb concentrations were determined by absorbance measurements at 340 nm using a molar absorptivity of 26,000 M⁻¹cm⁻¹ as provided by the manufacturer. DNA was quantified by absorbance measurements at 260 nm. Labeling ratios were determined by linear combination of the respective absorbance values of Tb and oligo within the Tb-oligo conjugates.

*Photophysical **properties.*** Absorption spectra (Lambda 35 UV/Vis System, PerkinElmer) and emission spectra (FluoTime 300, PicoQuant) were recorded in HEPES buffer (100 mM, pH 7.4) and purified water for Tb and Cy5.5 samples, respectively. Photophysical properties of Tb-donor and of the acceptor dyes Cy5.5 are illustrated in Figures 2A and 2B. Förster distance was 5.8 nm for Tb-to-Cy5.5 FRET. The emission spectra of Tb contain 4 bands and has a spectral overlapping with the absorbance spectra of Cy5.5. The emission spectra of Cy5.5 can be recorded at 716±20 nm.

***ssDNA-20b assays.*** The ligation mixture consisted of 2 Weiss U of T4 DNA ligase, 0.5 nM padlock probe (SEQ ID NO: 5), and an appropriate amount of the target DNA in 30 µL ligation buffer (buffer 1, 40 mM Tris-Cl, 10 mM MgCl₂, 0.5 mM ATP, pH 7.8) and was incubated at 37°C for 1 hr. And then 5 U of phi29 DNA polymerase and 0.5 mM dNTP being prepared in 60 µL phi29 polymerase buffer (buffer 2, 40 mM Tris-Cl, 50 mM KCl, 10 mM MgCl₂, 5 mM (NH₄)₂SO₄, pH 7.5) were added and incubated at 37°C for 3 hrs. The polymerization was terminated by incubation at 60°C for 10 mins. After that, 60 µL buffer 2 containing 5 nM Tb probe (SEQ ID NO: 9) and 5 nM Cy5.5 probe (SEQ ID NO: 7) was added into the solution and was incubated at room temperature (22 °C) for 30 min before measurements. The total reaction volume was 150 µL and 140 µL of sample was transferred and measured in black 96-well microtiter plates with an optimal working volume of 140 µL. PL decay curves were acquired on a fluorescence plate reader (Edinburgh Instruments) using 4000 detection bins of 2 µs integration time and nitrogen laser (MNL 100, LTB Lasertechnik Berlin) excitation (337.1 nm, 20 Hz). Optical transmission filter bandpass wavelengths were (494±10) nm (Semrock) for the Tb detection channel and (716±20) nm (Semrock) for the Cy5.5 detection channel. Time-gated (0.1 - 0.9 ms) PL intensity measurements were acquired on a KRYPTOR compact plus clinical fluorescence plate reader (Cezanne/Thermo Fisher Scientific) using the same Tb and Cy5.5 detection channel bandpass filters as mentioned above. In single sensor assay, each measurement consisted of 100 excitation flashes at 20 Hz repetition rate (5 s per measurement). For statistical analysis and the estimation of LODs all samples were prepared 3 times and measured in triplicates (n = 9) apart from the zero-concentration samples (without DNA targets), which were prepared 10 times and measured in triplicates (n = 30).
Figure 3 shows that sensitivity of the method for detecting a short single-stranded DNA can reach a limit of detection of 5 fM.

***Triplexed ssDNA assays.*** Measurements of three different ssDNAs (20a, 20b, and 21) were also performed. In the control measurements, only one of the three padlock probes was present in the sample, and in the FRET measurements, both the ssDNA and its respective padlock probe were present. All six samples (three controls and three samples with the three different ssDNA) followed the ligation step and polymerisation step, and then their respective Tb probes or dye probes were added separately to perform FRET.

Aggregates (flocs) in the samples after the generation of RCA products strongly suggested the process of RCA had successfully happened. Dye sensitization and Tb quenching by FRET could be clearly observed with all ssDNAs, and more importantly, the PL decay curves of dye detection channels showed strong distance dependence, where the ssDNA sensor with shorter Tb-to-dye distance showed much faster decay curves and vice versa.

**Temporal multiplexing:** All FRET assays were prepared in hybridization buffer (25mM HEPES buffer, 150mM NaCl, 10mM MgCl2, 0.1% BSA, pH7.4) and contained 100 µL of Tb-DNA at constant concentrations to which 50 µL of F1-DNA at different concentrations were added. The single sensor assays contained 3 nM of Tb-DNA and increasing concentrations of F1-DNA (from 0.5 nM to 3 nM), and all samples were prepared once. The triplexed assays contained three LTC-DNAs (3 nM of each Tb-DNA) and different concentrations of the three F1-DNAs (from 0.5 nM to 3 nM), and all samples were prepared once.

After sample preparation they were incubated in black 96-well microtiter plates for 30 min at room temperature (22 °C) with an optimal working volume of 150 µL. PL decay curves were acquired on a fluorescence plate reader (Edinburgh Instruments) using 4000 detection bins of 2 µs integration time and nitrogen laser (MNL 100, LTB Lasertechnik Berlin) excitation (337.1 nm, 20 Hz). Optical transmission filter bandpass wavelengths were (494±10) nm (Semrock) for the Tb detection channel, (716±20) nm (Semrock) for the F1 detection channel.

Figures 4A, 4B, 4C, 4D and 4E show the PL decay curves of the F1 detection channels with different lengths of dsDNAs. The long-lifetime parts of the decay curves strongly increase with increasing F1 probe concentrations for all dsDNAs, and furthermore, the decay curves show strong distance dependence where the dsDNA with shorter length shows much faster decay curves and vice versa.

Using the distinguishable decay curves of the sensitized F1 from different lengths of dsDNAs, also temporal multiplexing was performed. Three lengths of dsDNA which are 10 bps, 13 bps and 25 bps are chosen to show the possibility of temporal multiplexing. In the triplexed DNA assay, all Tb-DNA probes and different amounts of the F1-DNA probes were present into a single sample. We therefore prepared four different assays. The first three assays contained only one of the F1-DNA probes at increasing concentrations from 1 nM to 3 nM. The last assay contained the same increasing concentrations of all F1-DNA probes. When only one of the F1-DNA probes was present in the samples, it showed similar decay curves to the single sensor assays and when all three F1-DNA probes were present, the decay curves showed a linear combination of the three decay curves when only one F1-DNA probe was present. Three different time-gated windows (TGi: 0.02-1.5 ms, TGii: 2.5-5.0 ms, TGii: 6.0-8.0 ms) were selected to calculate the FRET ratio (time-gated PL intensities of the F1 channel were divided by that of the Tb channel) of all three energy donor-acceptor pair probes (Tb and F1 within distances of 10bps, 13bps, and 25 bps). These three pairs of probes showed different contributions in three time-gated windows (Figures 5A, 5B and 5C). More importantly, the FRET ratios of the samples when all three F1 probes were present overlaid with the FRET ratios summation calculated of the samples when only one of the F1 probes was present (Figures 5D, 5E and 5F), which meant we could simply deconstruct the contributions of all F1 probes in three TG windows and achieved temporal multiplexed determinations of all F1 probes.

## Claims

1. Kit for detecting or quantifying at least one nucleic acid target of at least 16 nucleotides, in particular of 18 to 40 nucleotides, by time-resolved Förster resonance energy transfer measurement combined with DNA-primed rolling circle amplification, in a sample obtained from a biological sample, said nucleic acid target being represented from its 5' end to 3' end by structure TGT5'-TGT3', wherein
- TGT5' is the part situated at 5' end of a nucleic acid target;
- TGT3' is the part situated at 3' end of a nucleic acid target,
said kit being constituted by:
(i) at least one padlock probe of at least 60 nucleotides, in particular of 70 to 120 nucleotides, represented from its 5' end to 3' end by structure PRB5'-LINK-PRB3', wherein
- PRB5' is a nucleic acid fragment of at least 8 nucleotides, in particular of 9 to 20 nucleotides, situated at 5' end of padlock probe, said PRB5' fragment having nucleic acid sequence complementary to that of at least a part of TGT5' fragment;
- PRB3' is a nucleic acid fragment of at least 8 nucleotides, in particular of 9 to 20 nucleotides, situated at 3' end of padlock probe, said PRB3' fragment having nucleic acid sequence complementary to that of at least a part of TGT3' fragment;
- LINK is a nucleic acid fragment situated between the fragments PRB5' and PRB3', of at least 44 nucleotides, in particular of 50-110 nucleotides,
the padlock probe number being equal to that of nucleic acid target(s) to be detected or quantified,
(ii) at least one pair of energy donor-acceptor probes, whose number is equal to that of nucleic acid target(s), in each pair:
- said energy donor probe named D-probe comprising an oligonucleotide named Nd-D of at least 8 nucleotides, in particular 10 to 30 nucleotides, Nd-D having nucleic acid sequence identical to that of a first part of padlock probe, said Nd-D oligonucleotide being labelled at 5' or 3' end by a luminescent material of long excited-state lifetime, as energy donor,
- said energy acceptor probe named A-probe comprising an oligonucleotide named Nd-A of at least 8 nucleotides, in particular 10 to 30 nucleotides, Nd-A having nucleic acid sequence identical to that of a second part of padlock probe, said Nd-A oligonucleotide being labelled at 5' or 3' end by a luminescent material, as energy acceptor,
- said energy donor having an average excited-state lifetime at least 10 times longer than any one of the energy acceptor,
(iii) optionally, a ligase,
(iv) optionally, a polymerase,
(v) optionally, reaction buffers.

2. Kit according to claim 1, wherein:
- nucleic acid sequence of PRB5' fragment is completely complementary to that of TGT5' of the nucleic acid target to be detected or quantified;
- nucleic acid sequence of PRB3' fragment is completely complementary to that of TGT3' of the same nucleic acid target.

3. Kit according to claim 1 or 2 for detecting or quantifying at least two nucleic acid targets, containing at least two padlock probes and at least two pairs of energy donor probe and energy acceptor probe, wherein:
- the energy donors of each energy donor probe are identical; and
- the energy acceptors of each energy acceptor probe are different each other.

4. Kit according to claim 1 or 2 for detecting or quantifying at least two nucleic acid targets, comprising at least two padlock probes and at least two pairs of energy donor probe and energy acceptor probe, wherein
- the energy donors of each energy donor probe are identical;
- the energy acceptors of each energy acceptor probe are also identical ;
- the distances between the energy donor and the energy acceptor of each pair of energy donor-acceptor probes are different.

5. Kit according to any one of claims 1 to 4, wherein the energy donor is chosen from the group comprising a lanthanide ion or a transition metal such as Ru, Ir, Os, Pt or Re, or a long-lifetime fluorophore such as fluorazaphores, or a long-lifetime nanoparticle such as semiconductor quantum dots or quantum rods or a combination thereof.

6. Kit according to claim 5, wherein the energy donor is a lanthanide ion chosen from Tb³⁺, Eu³⁺, Sm³⁺, Dy³⁺, Er³⁺, Tm³⁺ or Ho³⁺.

7. Kit according to any one of claims 1 to 6, wherein the energy acceptor is an organic fluorophore, such as pyrene-, naphthalene-, coumarin-, fluorescein-, rhodamine- or cyanine-based dyes, or a non-fluorescent dark quencher, or a polymeric or dendrimeric dye, or a nanoparticle such as semiconductor quantum dot or quantum rod, or a naturally occurring fluorophore such as fluorescent protein or light harvesting complex or a combination thereof.

8. Kit according to any one of claims 1 to 7, wherein the nucleic acid target is chosen from a microRNA, a siRNA, a ssDNA, a mixture thereof, or an SNP site.

9. Kit according to any one of claims 1 to 8, wherein a biological sample is chosen from a sample obtained from biological fluid, from an *in vitro* cell culture, from an *ex vivo* tissue, from plants, from yeast, from bacteria or from exosomes.

10. Kit according to claim 9, wherein the biological fluid is chosen from serum, inactivated serum, plasma, or blood.

11. Kit according to claim 9, wherein the sample of a tissue is a sample obtained by biopsy or during surgical operation.

12. Method for detecting or quantifying the amount of one or multiple nucleic acid targets of at least 16 nucleotides, in particular 18 to 40 nucleotides, in a sample obtained from a biological sample, said method comprising the steps of:
(i) Adding to said sample or a solution extracted from said sample, the padlock probe(s) of the kit as defined in claim 1, a ligase and a ligation buffer,
(ii) Adding to the mixture obtained at the end of step (i) a polymerase, dNTP and polymerase buffer to carry out a rolling circle amplification,
(iii) Adding to the mixture obtained at the end of step (ii), the pairs of energy donor-acceptor probes, whose number is equal to that of padlock probes(s),
(iv) Measuring photoluminescence emission intensities issued from energy donor and energy acceptor in a specific time window.

13. Method according to claim 12, wherein photoluminescence emission intensities issued from energy donor and energy acceptor are measured in a specific time window starting at a time point of at least 10 times of the excited-state lifetime of the acceptor after light excitation and having a temporal width of at least the excited-state lifetime of the energy acceptor.

14. Method according to claim 12 or claim 13 for detecting or quantifying, the amount of at least two nucleic acid targets in a sample, wherein at least two padlock probes and at least two pairs of energy donor probe and energy acceptor probe are used,
- the energy donors of each energy donor probe being identical;
- the energy acceptors of energy acceptor probe being also identical,
the photoluminescence emission intensity issued from each energy acceptor is measured in distinct specific time windows after light excitation.


SEQUENCE LISTING

<110>  Université Paris Sud
       CNRS
       Lumiphore Inc.

<120>  KIT AND METHOD FOR DETECTING OR QUANTIFYING ONE OR MULTIPLE
       NUCLEIC ACID TARGETS

<130>  IOB UPS RCP

<160>  18

<170>  PatentIn version 3.5

<210>  1
<211>  23
<212>  DNA
<213>  artificial sequence

<220>
<223>  ssDNA-20a

<400>  1
taaagtgctt atagtgcagg tag                                              23


<210>  2
<211>  23
<212>  DNA
<213>  artificial sequence

<220>
<223>  ssDNA-20b

<400>  2
caaagtgctc atagtgcagg tag                                              23


<210>  3
<211>  22
<212>  DNA
<213>  artificial sequence

<220>
<223>  ssDNA-21

<400>  3
tagcttatca gactgatgtt ga                                               22


<210>  4
<211>  79
<212>  DNA
<213>  artificial sequence

<220>
<223>  padlock ssDNA-20a

<400>  4
aagcacttta atatatgatg gaccgctcaa ttaactgaag atgccgaatt tttcaagagg      60

aaatgtctac ctgcactat                                                   79

<210>  5
<211>  79
<212>  DNA
<213>  artificial sequence

<220>
<223>  padlock ssDNA-20b

<400>  5
gagcactttg atatatgatg gaccgctagg attaactgag atgccgaatt tttcaagagg        60

aaatgtctac ctgcactat                                                     79


<210>  6
<211>  78
<212>  DNA
<213>  artificial sequence

<220>
<223>  padlock ssDNA-21

<400>  6
tgataagcta atatatgatg gaatcaagac aatattgttg atgccgaatt tttcaagagg        60

aaatgttcaa catcagtc                                                      78


<210>  7
<211>  20
<212>  DNA
<213>  artificial sequence

<220>
<223>  Cy5.5 probe

<400>  7
gatgccgaat ttttcaagag                                                    20


<210>  8
<211>  10
<212>  DNA
<213>  artificial sequence

<220>
<223>  Tb probe ssDNA-20a

<400>  8
attaactgaa                                                               10


<210>  9
<211>  13
<212>  DNA
<213>  artificial sequence

<220>
<223>  Tb probe ssDNA-20b

<400>  9

taggattaac tga                                                          13

<210>  10
<211>  18
<212>  DNA
<213>  artificial sequence

<220>
<223>  Tb probe ssDNA-21

<400>  10
aatcaagaca atattgtt                                                     18

<210>  11
<211>  10
<212>  DNA
<213>  artificial sequence

<220>
<223>  ssDNA-Tb (10bps)

<400>  11
attcggcatc                                                              10

<210>  12
<211>  13
<212>  DNA
<213>  artificial sequence

<220>
<223>  ssDNA-Tb (13bps)

<400>  12
taggattaac tga                                                          13

<210>  13
<211>  15
<212>  DNA
<213>  artificial sequence

<220>
<223>  ssDNA-Tb (15bps)

<400>  13
gaaaaattcg gcatc                                                        15

<210>  14
<211>  20
<212>  DNA
<213>  artificial sequence

<220>
<223>  ssDNA-Tb (20bps)

<400>  14
ctcttgaaaa attcggcatc                                                   20

<210>  15
<211>  25
<212>  DNA
<213>  artificial sequence

<220>
<223>  ssDNA-Tb (25bps)

<400>  15
tcgtgtctaa agtccgttag ctcat                                              25


<210>  16
<211>  20
<212>  DNA
<213>  artificial sequence

<220>
<223>  ssDNA-F1 (10 bps, 15bps, 20bps)

<400>  16
gatgccgaat ttttcaagag                                                    20


<210>  17
<211>  13
<212>  DNA
<213>  artificial sequence

<220>
<223>  ssDNA-F1 (13bps)

<400>  17
tcagttaatc cta                                                           13


<210>  18
<211>  25
<212>  DNA
<213>  artificial sequence

<220>
<223>  ssDNA-F1 (25bps)

<400>  18
atgagctaac ggactttaga cacga                                              25